# EUROPEAN PATENT APPLICATION

(11) **EP 1 913 907 A1**
(43) Date of publication of application: **23.04.2008**
(21) Application number: 06076908.0
(22) Date of filing: 20.10.2006
(51) Int. Cl.: A61F 5/058, B29C 33/38

(54) **A method and arrangement for shaping a skin contact orthesis, such as facial orthesis**

(71) Applicant: Academisch Ziekenhuis Maastricht, 6229 HX Maastricht (NL)
(72) Inventor: Boeckx, Willy Denis, 3210 Linden (BE); Colla, Carlo Gilbert Jozef, 3630 Maasmechelen (BE); van den Kerckhove, Eric André Maria Clément, 3001 Heverlee (BE); Gorissen, Kim Josephina, 6221 AT Maastricht (NL)
(74) Representative: van Loon, C.J.J.

(57) **Abstract**

A method for shaping a skin contact orthosis, such as a facial orthosis. The orthosis has a contact surface which can be positioned against the skin of at least a part of the body of an individual having an anatomical deformation in that part of the body. Shape data is inputted to a data processing unit. The shape data represents a current shape of at least that part of the body of said individual. Also, data representing parameter values related to a desired shape of at least that part of the body of the individual is inputted to the data processing unit. The data processing unit determines from the shape data and the input data, a surface shape of said contact surface. The contact surface of the skin contact orthosis is formed by an orthosis forming unit to the surface shape under control of the data processing unit.

## Description

### FIELD AND BACKGROUND OF THE INVENTION

The invention relates to a method for shaping a skin contact orthosis, such as a facial orthosis. The invention further relates to a method for treating an anatomical deformation, e.g. in the face of an individual. The invention further relates to an arrangement for shaping a skin contact orthosis. The invention also relates to a computer program product, to an orthosis forming unit, to a data processing unit and to a skin contact orthosis.

In the art of facial reconstruction, it is known to use a rigid facial mask, from hereon referred to as a facial orthosis, to remodel the shape of the facial skin or the structures, e.g. bones, under the facial skin. The facial mask is made by taking a negative plaster mould, from the face of the individual to be treated. From the negative plaster mould, a positive tangible model of the face in its current state is made. Typically, the tangible model is made of plaster or clay. The tangible model is subsequently modified to a desired shape of the face, by manually scraping material off at suitable locations. For example, the facial mask may be used to treat hypertrophic scars, and material may be removed from the model at the locations of the hypertrophic areas in order to make a facial mask which exerts a (mild) pressure on the skin at the hypertrophic areas. Once the tangible model has a suitable shape, the mask is formed with a facial contact area complementary to the shape of the tangible model, by vacuum moulding the mask material using the tangible model as a mould. During the treatment, a gradual change of the face from its current shape into the ideal shape is obtained by scraping more material from the positive tangible model at the desired locations and again vacuum moulding a mask.

However, a disadvantage of these known facial masks is that manufacturing of the facial mask is complex and expensive because the tangible model is made and modified manually. Furthermore, during treatment a large amount of storage space is required to store the models, especially in case a large number of individuals is treated.

### SUMMARY OF THE INVENTION

It is a goal of the invention to provide a method for shaping a skin contact orthosis, such as a facial orthosis, which is less complex and expensive. Therefore, according to a first aspect of the invention, a method according to claim 1 is provided.

Such a method allows a less complex and expensive shaping of the skin contact orthosis, because data representing the current shape of the part of the body and data representing a desired shape of that part of the body are inputted to the data processing unit and the data processing unit controls the orthosis forming unit to form the contact surface of the skin contact orthosis to the surface shape. Accordingly, the need for a manual preparation of a plaster model, and the associated complexity and expenses, is obviated.

Furthermore, since the orthosis forming unit is controlled by the data processing unit, the need to store a number of plaster models is obviated as well, since the orthosis forming unit is controlled by the data processing unit to form the contact surface. Accordingly, the data processing unit can determine a shape for a specific individual and control the orthosis forming unit correspondingly.

According to a second aspect of the invention, a method for treating an anatomical deformation according to claim 14 is provided.

According to a third aspect of the invention, an arrangement according to claim 16 is provided.

According to a fourth aspect of the invention, a computer program product according to claim 17 is provided.

According to a fifth aspect of the invention, an orthosis forming unit according to claim 18 is provided.

According to a sixth aspect of the invention, a data processing unit according to claim 19 is provided.

According to a seventh aspect of the invention, an orthosis according to claim 20 is provided.

These and other aspects of the invention will be apparent from and elucidated with reference to the embodiments described hereinafter. Specific embodiments of the invention are set forth in the dependent claims.

### BRIEF DESCRIPTION OF THE DRAWINGS

Further details, aspects and embodiments of the invention will be described, by way of example only, with reference to the drawings.

FIG. 1 schematically shows an example of an embodiment of an arrangement for shaping a skin contact orthosis according to the invention.

FIG. 2 schematically shows a sectional view of an example of an embodiment of a skin contact orthosis according to the invention.

FIG. 3 schematically shows a sectional view of an example of an embodiment of an adjustable model according to the invention.

FIG. 4 schematically shows a detailed view of the example of FIG. 3.

### DETAILED DESCRIPTION

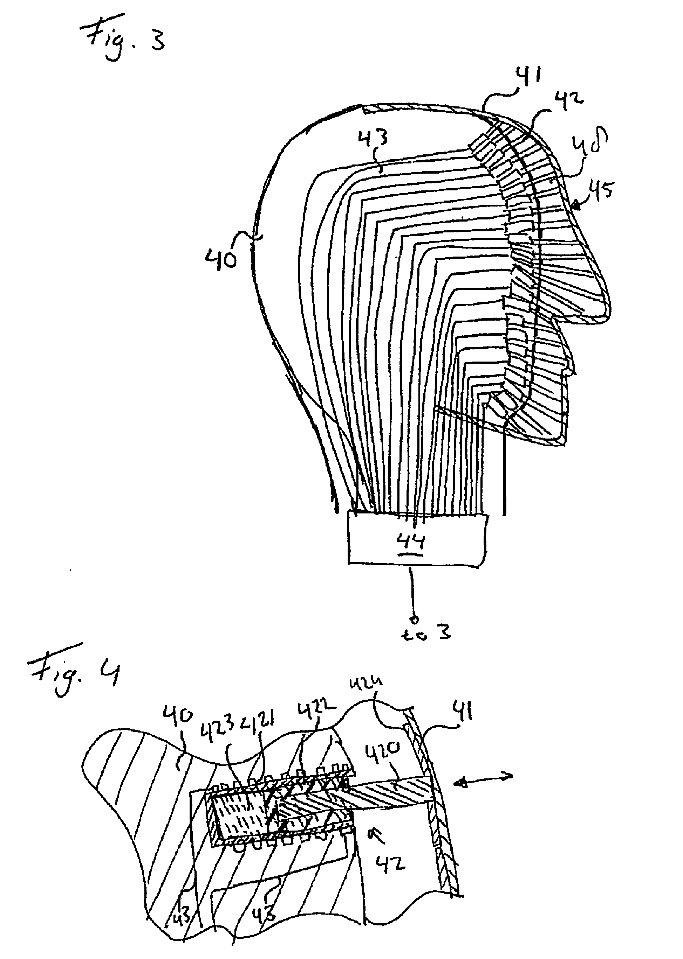

The example of an arrangement 1 shown in FIG. 1 includes a sensor unit 2, and a data processing unit 3 connected to the sensor unit 2. The sensor unit 2 can obtain information about the current shape of (at least a part of) the body, in this example (at least a part of) the face, of an individual and transmit the information to an input of the data processing unit 3, from hereon referred to as the sensor input 30.

The current shape may, for example, be determined prior to a treatment or be determined after treatment has started. The sensor unit 2 may be a contact less sensor unit which obtains the shape information without physical contact with the face. Thereby, the shaping of the orthosis is less invasive to individual.

For instance in this example, the sensor unit 2 includes an optical imaging system with a number of cameras or image capturing devices 20, 21 which are able generate a stereoscopic image of the face. The current shape of the face can be determined by the processing unit 3 from the stereoscopic image received at the sensor input 30. It should be noted that determining the shape of an object from a stereoscopic image is known in the art of stereoscopic imaging, for example of United States patent application publication US 2005-151839 A1, relevant parts incorporated herein by reference, and for the sake of brevity is not described in further detail.

However, the sensor 2 may be implemented in a different manner. The arrangement may for example include a single optical sensor which generates an image of the face and a light source which projects a line-shape beam of light on the face and which moves over the face. From the displacement of the beam of light in the image generated by the sensors, the shape of the face can be determined, as is described in United States patent application publication US 2006-0055943, relevant parts incorporated herein by reference, and for the sake of brevity is not described in further detail. A second input of the data processing unit 3, from hereon referred to as the operator input 31, is connected to a user-interface 6, in this example a suitably programmed general purpose computer 60 provided with a display 61, at which the information about the current shape of the part of the body, in this example the face, can be outputted in a for humans perceptible form. An operator, e.g. an orthopaedic engineer, can perceive the information and based thereon determine a desired shape of the part of the body, in this example the face or a part of the face. The operator may subsequently input via the user interface 6 to the data processing unit 3 information about a desired shape of the part of the body, in this example the face. Based on the data inputted by the operator, the data processing unit 3 controls the shape to which the skin contact orthosis, in this example the facial orthosis 7, is formed by an orthosis forming unit 4.

It will be appreciated that the operator, and the user interface 6, need not be present at the location where the current shape of the face is determined by the sensor unit 2. The information about the current shape of the face may, for instance, be transmitted from the sensor unit 2 to the user interface 6, e.g. via the internet. This provides the advantage that it is possible that one operator may, if so desired, process information about current shapes of faces from a plurality of sensor units which may be located at various different locations, without requiring the operator to travel to these different locations. The sensor unit may also be arranged to transmit information about the current shape of the face to a user interface of a plurality of user interfaces, e.g. depending on operator workload or expertise.

It will also be appreciated that the operator, and the user interface 6, need not be present at the location where the forming unit 4 is present. A signal representing the shape to which the facial orthosis is to be formed may, for instance, be transmitted from the user interface 6 to the forming unit 4, e.g. via the internet. This provides the advantage that it is possible that the manufacture of the orthosis is performed at a location that may be chosen independent of the location of the user interface or the sensor unit. Further, a single forming unit 4 may be arranged to, if so desired, receive signals representing the shapes to which the facial orthoses are to be formed from a plurality of user interfaces so that manufacture of orthoses can be performed centralised for a plurality of user interfaces, thus minimising production costs. The user interface may also be arranged to transmit the signals representing the shape to which the facial orthosis is to be formed to a forming unit of a plurality of forming units, e.g. if a large manufacturing capacity is required for that user interface.

It will be appreciated that the processing unit 3 may be present at the location of the sensor unit 2, at the location of the user interface 6, at the location of the forming unit 4, at a remote location or distributed among two or more of these locations.
It should be noted that the desired shape may be the desired final shape of the part of the body, in this example the face, after treatment, which from hereon is referred to as the *'ideal shape',* or the desired shape after a non-final stage of treatment, from hereon referred to as the *'intermediate shape'.* For example, the operator may input at certain times during the treatment information about an intermediate shape deemed suitable by the operator to gradually transform the shape of the face from the current shape to the ideal shape, for instance after the shape of the face at that time, i.e. after a certain period of treatment, is determined from facial data obtained with the sensor.

However, it is also possible that the operator inputs the ideal shape and the data processing unit 3 determines from the differences between the ideal shape and the current shape intermediate shapes suitable to gradually transform the face from the current shape to the ideal shape.

The operator may input at the user interface 6 parameter values related to a desired shape of at least a part of the face of the individual. The operator may input information which defines the shape directly, e.g. as coordinates in a three-dimensional space. For example, the data processing unit 3 may generate a three dimensional computer model of the current shape of the face from the facial data and output at the user interface 6 a two dimensional projection of the model. The operator may then from the projection determine a suitable desired shape. The operator may for example adjust the shape defined by the model by inputting at the user-interface suitable commands. However, the operator may also input information which defines the shape indirectly, for example in terms of the amount and location of pressure to be exerted by the orthosis on the facial skin the operator deems appropriate to bring the face into the desired shape. In such case, the data processing unit 3 may be provided with a mathematical algorithm which, based on e.g. the material parameters of the tangible model and the elastic parameters of the skin, determines a shape of the tangible model suitable to exert the given amount of pressure at the desired location.

The orthosis forming unit 4 may be implemented in any manner suitable to form the skin contact orthosis, and for example include an adjustable tangible model of the desired shape of the face. The tangible model may for example be a negative, tangible model of the face which is used as a mould to shape the facial orthosis 7. For example, the facial orthosis 7 can be pressed on the mould with an outer surface 77 of the orthosis (which faces away from the contact surface 76 of the orthosis) oriented towards the negative model. In the example of FIG. 1, the orthosis forming unit 4 is a positive, adjustable, tangible model of the face, on which a facial orthosis 7 can be positioned. The facial orthosis has, as shown in FIG. 2, an at least partially adjustable contact surface 76 for being in use positioned against the facial skin of an individual having an anatomical deformation in the face. The contact surface 76 is positioned towards the positive tangible model. By exerting on the facial orthosis 7 a force towards the positive model, the contact surface 76 of the facial orthosis 7 will shaped into a suitable form. More in particular, the contact surface 76 will conform to the contour of the part of surface 45 of the model against which the contact surface 76 lies and hence obtain a suitable shape. In this example, the orthosis forming unit 4 exerts a force on the facial orthosis 7 by means of vacuum, however, the force may also be exerted by exerting a pressure force on the facial orthosis 7, e.g. by pressing towards the model on a non-contact surface 77 of the orthosis 7.

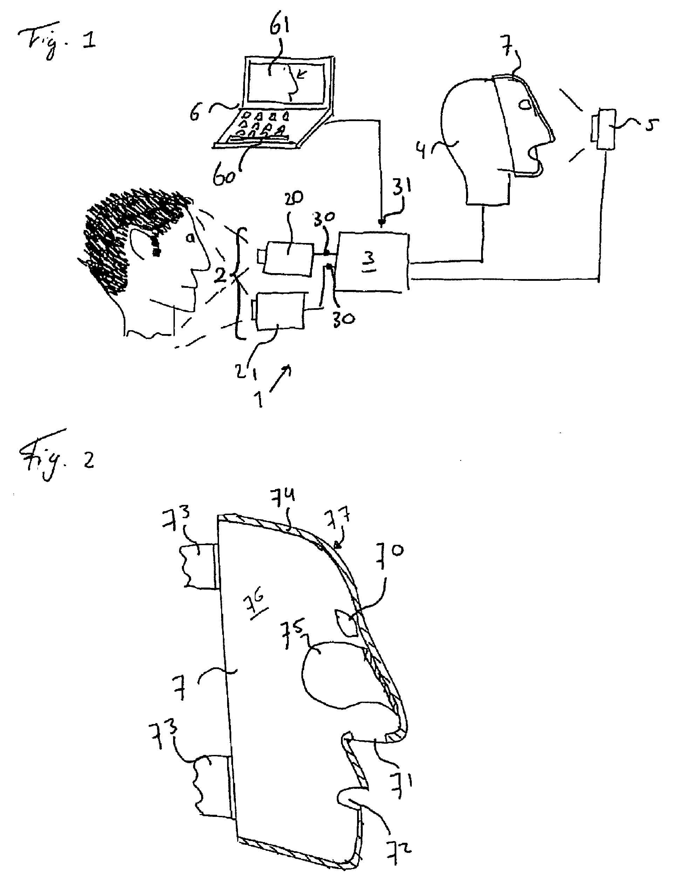

As shown in FIGs. 3 and 4, the tangible model may have a flexible surface 45 which determines the shape of the contact surface 76 of the orthosis 7 and may therefore be referred to as the orthosis forming surface 45. By adjusting the shape of the contact surface 45, for example as described below with reference to FIGs. 3 and 4, the facial orthosis 7 can be shaped into a suitable form, without the necessity of making a new model for each patient or having to adjust a plaster model by removing material. Hence, the facial orthosis can be manufactured in a less complex and expensive manner. Furthermore, the tangible model can be adjusted to the face of a particular individual, and hence the need to store a model for each individual is obviated. If so desired, information defining the current and/or ideal shape of the face of an individual may be stored, e.g. in a computer memory of the processing unit 3.

In the example of FIG. 1, the surface 45 of the orthosis forming unit 4 is controlled by the data processing unit 3 to follow a contour which is a positive of the desired shape of the contact surface 76 of the orthosis 7. As shown in FIG. 3, the orthosis forming unit 4 may for example include a core or base 40 which is provided at a side with a flexible membrane 41. The membrane 41 can be formed into a suitable shape. In FIGs. 3 and 4 for example, the membrane 41 can be formed into the desired shape by varying the spacing between the core or base 40 and the flexible membrane 41. The membrane 41 can be moved towards or away from the core or base 40 by means of a plurality of movable spacers 42. The movable spacers 42 can be controlled separately by the data processing unit 3. In FIG. 3, for instance, the movable spacers 42 are connected with control lines 43 to a routing device 44 which routes control signals from the data processing unit 3 to a suitable one of the control lines 43.

The orthosis forming unit 4 in the example of FIG. 3 may further include a suitable force exerting unit. For example, the membrane 41 may be provided with small perforations or suction holes, and a pumping unit may be connected to the space 48 held by the spacers 42 between the flexible membrane 45 and the core 40, to generate a vacuum (or at least a low pressure in the space 48 which is lower than the outside pressure). The vacuum or low pressure is then transferred into suction via the suction holes and a facial orthosis 7 positioned on the orthosis forming unit 4 will be sucked onto the orthosis forming unit 4 and be forced to conform to the shape of the flexible membrane 45 and hence be formed into the desired shape.

In the example of fig, 3 and 4, an arrangement of spacers 420 is provided. As shown in more detail in FIG. 4, the controllable spacers 42 may include a shaft 420 which is movable relative to the core 40 and which supports the flexible membrane 45 at a supporting end 424 which lies adjacent to and in contact with the flexible membrane 45. Thus, when the shaft is moved to a position, the flexible membrane 45 flexes correspondingly, and when the shaft is kept in position, the flexible membrane maintains shape. Accordingly, by moving the spacers 420 such that their supporting ends 424 are positioned corresponding to the desired shape, the shape of the model can be adjusted. In the example of FIG. 4, the shaft 420 is provided at the protruding end 424 with a plate-shape tile which, in this example, extends substantially perpendicular to the longitudinal axis of the shaft 420 and supports the flexible membrane over a larger area. The supporting ends 424 of the arrangement hence form a pattern of supporting tiles each of which can be moved in a direction perpendicular to the respective tile. The tiles and/or shafts may be oriented such that the arrangement itself follows a general facial contour and by moving the shafts 420 the shape can be adjusted to the shape of the face of a specific individual.

The movement of the shaft 420 may be driven and the position of the shafts be controlled in any manner suitable for the specific implementation. The shaft 420 may for instance be part of a linear motor. In FIG. 4, for instance, the shaft 420 is part of a magnetic linear motor. The shaft 420 can slide in and out of a recess 423 and is made of a magnetic material. Electro-magnets 421 are positioned near the recess 423. A magnetic field can be generated in the recess 423 by applying a current to the electromagnet 421, e.g. via the control line 43. By means of the magnetic field, the magnetic shaft 420 can be driven in or out of the recess 423 in a controllable manner.

FIG. 2 shows an example of a skin contact orthosis 7, in this example a facial orthosis. The example includes a mask 74 which has an inner surface 76 defining a cavity. During treatment, the mask 74 will be positioned on the face of an individual such that the face of the individual to be treated is placed in the cavity with the inner surface 76 positioned in contact with and lying against at least a part of the facial skin. The mask 74 is provided with suitable passages 70-72 for e.g. the mouth, nose and eyes of the individual. The mask 74 can be held in position with elastic bands 73 which may extend from a side of the mask 74 along the head to another side of the head.

The mask 74 may be of a material with a stiffness at room temperature sufficient to exert a pressure onto at least a part of the face. Without whishing to be bound to any theory, it is found that the skin is sensitive to pressure and that by exerting, during a prolonged time, a pressure the structure of the skin can be remodelled. A suitable period of time has been found to be between about 12 and 24 months, as an average a period of 16 months has been found to be suitable. In particular, it is found that the thickness of scars, such as hypertrophic scars caused by burns, trauma or surgical incisions can be reduced by exerting pressure.

The material of the mask 74 may be a thermoplastic material which softens sufficiently upon heating to adjust the shape of the mask. For example, the thermoplastic material may soften sufficiently to be formed by vacuum moulding Thereby, the life time of the facial orthosis can be prolonged, since the facial orthosis can be adjusted during treatment in a simple manner, e.g. in the example of FIG. 1 by controlling the shape of the flexible membrane to an adjusted shape and forming the facial orthosis on the tangible model. The mask 74 may be pre-formed prior to forming the mask 74 to the desired shape with the arrangement 1, and for example be formed to a general face-like contour. The mask 74 may for example be of a sheet-like material which is formed in a suitable three dimensional shape by the arrangement 1 (and may optionally be pre-formed).

The material of the mask 74 may be gas-permeable, and optionally liquid permeable, and/or be transparent to visible light. In the example of FIG. 2, the mask 74 is made of a gas and liquid permeable material and the inner surface 76 is locally covered with a patch 75 of a material which is gas-permeable but liquid and vapor-impermeable, e.g. silicone or an other occlusive material. Without whishing to be bound to any theory, this allows the skin to breath, while increasing the amount of moisture in the skin. Thereby, the height and/or structure of scars can be reduced. A further reduction of the height and/or improvement of the structure of scars can be obtained by exerting at the location of the patch 75 a (mild) pressure by means of the mask 74. A suitable amount of pressure is found to be a pressure of equal to or larger than 25 mm Hg.

In addition, it is also possible that the skin contact orthosis includes a drug delivery system which provides a suitable type of drug to the scarred area. For example, the surface of the mask may be provided with a coating which releases a drug. Also, a fluid supply structure mouthing near the patch may be provided, which during use provides a fluid which contains a suitable drug.

The arrangement of FIG. 1 may be used to treat an anatomical deformation in the face of an individual. In this respect, an anatomical deformation may for example include a deformation of a type requiring medical supervision, such as severe scars, burns, surgical incisions and post traumatic skin lesions, scars and deformations with cosmetic deformations. Accordingly, the treatment may be a medical treatment or a cosmetic treatment. Without whishing to be bound to any theory, a water impermeable, gas permeable membrane, such as the patch 75 in the example of FIG. 2, is believed to restore moisture in the skin, plumping indentations and smooth the appearance since it acts as the epidermis. Furthermore, it is believed that covering the facial skin at least partially with a water impermeable, gas permeable membrane results in an increased production of good quality collagen and elastin and increases the aesthetic appearance of the skin by improving its texture. Accordingly, the facial orthosis may be used in a cosmetic treatment.

During treatment, the facial orthosis 7 is placed on the face of the individual to be treated in such a manner that the facial orthosis 7 exerts the desired type of influence on the facial skin and/or bone structure. For example, the facial orthosis 7 may (locally) exert a pressure e.g. to remodel the bone structure or to reduce the formation of hypotrophic scars. However, the orthosis may also influence the face in another manner and for example (at least partially) inhibit the transport of moisture away from the skin surface or release compounds which e.g. influence the face or relieve pain, or any other suitable type of influence. After a suitable period of use of the skin contact orthosis by the individual, an adjusted skin contact orthosis may then be provided with a contact surface having an adjusted shape, for example to ensure that the amount of exerted pressure remains sufficient to remodel the bone and/or to treat the skin. The adjusted facial orthosis may for example be obtained by adjusting the current facial orthosis or by manufacturing a new orthosis with an adjusted shape.

In the foregoing it was already described that exerting a pressure to at least a part of the body by means of the mask 74 may provide a further reduction of the height and/or improvement of the structure of scars. During the progress of the method for treating the anatomical deformation, the height of a scar reduces. This causes the mask to make more even contact with the face over a larger area. Thus, the contact pressure at the position of the scar will reduce when the height of the scar reduces. Reduction of the contact pressure at the scar makes the method less effective. As already described, a pressure of equal to or larger than 25 mm Hg is preferred for effective treatment of the anatomical deformation.

In an advanced embodiment of a method for shaping a skin contact orthosis and/or treating an anatomical deformation using the mask 74 it is of benefit to measure the pressure exerted by the mask in use, e.g. continuously or at intervals, during the treatment. Thereto a pressure sensor, or a plurality of pressure sensors, may be placed between the mask and the face. When a plurality of sensors is used the respective pressures exerted to each individual pressure sensor may be measured e.g. simultaneously or sequentially. Preferably a sheet-shaped pressure sensor is used between the mask and the face. Those skilled in the art will appreciate that it is possible to calculate a change in the pressure exerted by the mask due to insertion of the pressure sensor.

The measurement of the pressure exerted by the mask can be used to determine whether or not the adjusted facial orthosis should be provided or the current facial orthosis suffices. If a measured pressure at a predetermined location between the face and the mask is below a predetermined threshold pressure, e.g. below 15 mm Hg, preferably below 25 mm Hg, it may be decided to provide the adjusted facial orthosis.

Measuring and checking the pressure at at least one location between the orthosis and the body may also be performed automatically, e.g. by a verification unit. The verification unit is thereto arranged to receive at least one pressure measurement signal from at least one pressure sensor between the orthosis and the body, compare the measured pressure associated with the received pressure measurement signal with the predetermined threshold pressure and determine, on the basis of the comparison whether an adjusted orthosis should be provided, and optionally indicate this e.g. to the operator. In an advanced embodiment, the verification unit estimates, on the basis of a pressure measurement, when the pressure exerted by the orthosis to the body, at that location, will become smaller than the threshold pressure. The verification unit may estimate, on the basis of at least two pressure measurements, taken at the same location between the orthosis and the body, but at different moments in time, when the pressure exerted by the orthosis to the body, at that location, will become smaller than the threshold pressure. Thus, the verification unit may estimate when the adjusted orthosis will be required. This provides the advantage that the adjusted orthosis may be manufactured beforehand, so that the adjusted orthosis is already present when needed, or that an appointment may be planned beforehand to have the current orthosis adjusted.

In another advanced embodiment the data processing unit 3 is arranged to determine from the pressure measurement and the ideal shape, and optionally the current shape, an intermediate shape of the orthosis suitable to gradually transform the face from the current shape to the ideal shape.

In the foregoing specification, the invention has been described with reference to specific examples of embodiments of the invention. It will, however, be evident that various modifications and changes may be made therein without departing from the broader spirit and scope of the invention as set forth in the appended claims. For example, the facial mask 74 may be provided with more than one patch 75 and the patch may be at other locations than shown in the example. Furthermore, the adjustable tangible model 4 may be implemented in another manner and e.g. form a part of an injection mould or otherwise.

In the examples a facial orthosis has been described. It will be appreciated that a method for shaping a skin contact orthosis and/or treating an anatomical deformation according to the invention can also be applied to other body parts. A skin contact orthosis can also be used e.g. to treat an anatomical deformation, such as severe scars, burns, surgical incisions and post traumatic skin lesions, scars and deformations with cosmetic deformations, on the hand of an individual

The invention may also be implemented in a computer program for running on a computer system, at least including code portions for performing steps of a method according to the invention when run on a programmable apparatus, such as a computer system or enabling a programmable apparatus to perform functions of a device or system according to the invention. Such a computer program may be provided on a data carrier, such as a CD-rom or diskette, stored with data loadable in a memory of a computer system, the data representing the computer program. The data carrier may further be a data connection, such as a telephone cable or a wireless connection.

Also, the invention is not limited to physical devices or units implemented in non-programmable hardware but can also be applied in programmable devices or units able to perform the desired device functions by operating in accordance with suitable program code. Furthermore, the devices may be physically distributed over a number of locations, while functionally operating as a single arrangement. For example, in the example of FIG.1, the data processing unit 3 may be connected with the sensor input 30 to a source of data, i.e. the sensors 2, at a location different from the location to which the operator input 31 is connected. For example, the sensors 2 may be present at the practice of a physician treating the individual, such as a plastic surgeon, burn surgeon, dermatologist, orthopaedic surgeon or otherwise, and the user interface 6 may be present in the working space of an orthopaedic engineer.

Also, devices functionally forming separate devices may be integrated in a single physical device. For example, in the example of FIG. 1 the data processing unit 3 may be included in the general purpose computer 60.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. A method for shaping a skin contact orthosis, such as a facial orthosis, including a contact surface for being in use positioned against the skin of at least a part of the body of an individual having an anatomical deformation in that part of the body, the method including:
inputting to a data processing unit shape data about a current shape of at least that part of the body of said individual;
inputting to the data processing unit input data representing parameter values related to a desired shape of at least that part of the body of the individual;
determining by the data processing unit, from the shape data and the input data, a surface shape of said contact surface;
forming the contact surface of the skin contact orthosis by an orthosis forming unit to the surface shape under control of the data processing unit.

2. A method according to claim 1, wherein:
the orthosis forming unit includes an adjustable tangible model; the method further including adjusting the tangible model under control of the data processing unit to have a model shape corresponding to the desired shape; and
said contact surface of the skin contact orthosis is formed in accordance with the model shape.

3. A method according to claim 2, wherein said tangible model is a mould and forming the contact surface includes moulding the skin contact orthosis with the mould wherein adjusting the tangible model under control of the data processing unit includes:
adjusting a mould surface of the mould to have said model shape.

4. A method according to any one of the preceding claims, wherein said skin contact orthosis has an at least partially adjustable part which defines said contact surface.

5. A method according to claim 4, wherein said adjustable part is made of a thermoplastic material and forming said contact surface includes heating at least said adjustable part to a temperature sufficient to form the thermoplastic material and forming the heated thermoplastic material to the desired shape.

6. A method according to any one of the preceding claims, further including obtaining with a sensor said shape data and, optionally, generating a digital image or digital model of at least a part of the body of the individual from the shape data.

7. A method according to claim 6, further including: outputting the shape data at a user interface, and inputting at said user interface said parameter values; which user interface is connected to the data processing unit.

8. A method according to any one of the preceding claims, further including:
providing an adjusted skin contact orthosis with a contact surface having an adjusted shape, after a period of use of the skin contact orthosis.

9. A method according to any one of the preceding claims, further including:
measuring a pressure exerted by at least a part of the contact surface of the skin contact orthosis to at least a part of the body of the individual during use of the skin contact orthosis.

10. A method according to claim 8 and 9, further including:
providing the adjusted skin contact orthosis with the contact surface having the adjusted shape when the measured pressure is smaller than a predetermined threshold pressure.

11. A method according to claim 9, further including:
estimating, on the basis of the measured pressure, at what moment in time the pressure exerted by at least that part of the contact surface of the skin contact orthosis to at least that part of the body of the individual during use of the skin contact orthosis will be smaller than a predetermined threshold pressure.

12. A method according to any one of the preceding claims for shaping a facial orthosis, including a contact surface for being in use positioned against the facial skin of an individual having an anatomical deformation in the face, the method including:
inputting to a data processing unit facial shape data about a current shape of at least a part of the face of said individual;
inputting to the data processing unit input data representing parameter values related to a desired shape of at least a part of the face of the individual;
determining by the data processing unit, from the facial shape data and the input data, a surface shape of said contact surface;
forming the contact surface of the facial orthosis by an orthosis forming unit to the surface shape under control of the data processing unit.

13. A method for treating an anatomical deformation in a part of the body of an individual, including:
providing a skin contact orthosis obtainable with a method according to any one of the preceding claims and positioning the skin contact orthosis against the skin of that part of the body of said individual during a period of time.

14. A method for treating an anatomical deformation in the face of an individual, including:
providing a facial orthosis obtainable with a method according to any one of claims 1-12, and positioning the facial orthosis against the facial skin of said individual during a period of time.

15. An arrangement for shaping a skin contact orthosis, such as a facial orthosis, including a contact surface for being in use positioned against the skin of at least a part of the body of an individual having an anatomical deformation in that part of the body, said arrangement including:
a first input for receiving shape data about a current shape of that part of the body of said individual;
a second input for receiving input data representing parameter values related to a desired shape of at least that part of the body of the individual;
a data processing unit connected to said first input and said second input, for determining from the shape data and the input data, a surface shape of said contact surface of the skin contact orthosis; and
an orthosis forming unit for forming the contact surface of the skin contact orthosis, said orthosis forming unit including a control input connected to the data processing unit, for setting the orthosis forming unit by the data processing unit in accordance with the suitable shape.

16. An arrangement according to claim 15 for shaping a facial orthosis including a contact surface for being in use positioned against the facial skin of an individual having an anatomical deformation in the face, wherein:
the first input is arranged for receiving facial shape data about a current shape of at least a part of the face of said individual;
the second input is arranged for receiving input data representing parameter values related to a desired shape of at least a part of the face of the individual;
the data processing unit is arranged for determining from the facial data and the input data, a surface shape of said contact surface of the facial orthosis; and
the orthosis forming unit is arranged for forming the contact surface of the facial orthosis.

17. A computer program product including program code for performing a method when run on a programmable apparatus, said method including:
receiving shape data about a current shape of at least a part of a body of an individual;
receiving data representing parameter values related to a desired shape of at least that part of the body of the individual;
determining from the shape data and the input data, a suitable shape of said contact surface; and
controlling an orthosis forming unit to form a contact surface of a skin contact orthosis in accordance with the suitable shape.

18. An orthosis forming unit for an arrangement according to claim 16.

19. A data processing unit for an arrangement according to claim 16.

20. A skin contact orthosis, such as a facial orthosis, for use in, or obtainable by, a method according to any one of claims 1-14.
